# EUROPEAN PATENT APPLICATION

(11) **EP 1 977 793 A1**
(43) Date of publication of application: **08.10.2008**
(21) Application number: 06843285.5
(22) Date of filing: 26.12.2006
(51) Int. Cl.: A63B 24/00, A63B 23/18, A61M 16/00, A61B 5/08

(54) **RESPIRATION TRAINING MACHINE FOR SIMPLY JUDGING RESPIRING STATE AND RESPIRATION TRAINING PROGRAM PRODUCT**

(30) Priority: 20.01.2006 JP 2006012881
(71) Applicant: Omron Healthcare Co., Ltd., Kyoto 615-0084 (JP); Jichi Medical University, Shimotsuke-shi, Tochigi 329-0498 (JP)
(72) Inventor: SHIRASAKI, Osamu c/o OMRON HEALTHCARE CO., LTD., Kyoto-shi, Kyoto 615-0084 (JP); SHIGA, Toshikazu c/o OMRON HEALTHCARE CO., LTD, Kyoto-shi, Kyoto 615-0084 (JP); NAKASE, Yuzo c/o OMRON HEALTHCARE CO., LTD., Kyoto-shi, Kyoto 615-0084 (JP); KOBAYASHI, Hideyuki c/o OMRON HEALTHCARE CO., LTD., Kyoto-shi, Kyoto 615-0084 (JP); KARIO, Kazuomi c/o JICHI MEDICAL UNIVERSITY, Shimotsuke-shi, Tochigi 329-0498 (JP)
(74) Representative: Wilhelms · Kilian & Partner Patentanwälte
(86) International application number: PCT/JP2006/325901
(87) International publication number: WO 2007/083493

(57) **Abstract**

A breathing exerciser (100) includes: a display unit (4) for displaying information for guiding an exercise pattern of breathing to a user; and a state switch (21.5) which is capable of detecting an operation by the user and which is for accepting an operation according to a breathing state. By this, the breathing exerciser (100) distinguishes during an exercise period during which the exercise pattern is guided, whether it is an exhalation state or an inhalation state, based on a signal from the state switch (21.5).

## Description

### TECHNICAL FIELD

The present invention relates to a breathing exerciser and a breathing exercise program produce, and more particularly, to a breathing exerciser and a breathing exercise program product that guide an exercise pattern of breathing.

### BACKGROUND ART

It has been verified that slow deep breathing leads to suppression of autonomic nerves, providing an effect of decreasing blood pressure. For example, the document "Slow Breathing Improves Arterial Baroreflex Sensitivity and Decreases Blood Pressure in Essential Hypertension" (hereinafter, referred to as the "Non-Patent Document 1 ") by Chacko N.Joseph, et al., in "Hypertension, October 2005", Volume 46, pp. 714-718, published by the American Heart Association, can be a reference document. Therefore, conventionally, as autonomic nervous system exercise methods and biofeedback, studies have been conducted. Also, a number of breathing exercisers for that have been proposed.

Japanese Patent Application Laid-Open No. 62-277976 (hereinafter, referred to as the "Patent Document 1 ") has disclosed an invention related to an abdominal breathing exercising apparatus in which a sensor that detects movement of the abdominal cavity caused by subject's abdominal breathing is placed on his/her abdomen, a predetermined ideal breathing exercise pattern is generated, an actual breathing pattern is compared with the ideal breathing exercise pattern to determine the degree of matching, and a result of the determination is informed by sound or photoelectric display.

Japanese Patent Application Laid-Open No. 2002-301047 (hereinafter, referred to as the "Patent Document 2") has disclosed an invention related to a breathing induction apparatus including a breathing detecting means of detecting breathing of a living body, in which breathing information is extracted from a detected breathing signal, the breathing information is compared for determination with target breathing pattern information to be induced, and a stimulus signal to be provided to the living body is controlled by a correction value which is based on a difference obtained by the comparison. For the breathing detecting means, there are exemplified one of a type that is placed on the abdomen or chest of a living body to detect a change in trunk length associated with breathing, one of a type that is attached near the nostril to measure a change in temperature, and one of a type that measures a change in airflow rate with a mask-type or mouthpiece-type instrument.

It has become clear that respiratory standstill during sleep due to airway obstruction or autonomic nervous system abnormalities, which is a so-called "Sleep Apnea Syndrome (SAS)", not only simply reduces sleep quality, causing drowsiness during daytime active hours but also promotes hypertension and thereby induces harmful blood pressure fluctuations, which becomes a cause of many serious diseases such as heart diseases and brain diseases.

As treatment approaches for the SAS, there have been proposed an apparatus (CPAP) that delivers a positive pressure of air to the obstructed airway, a surgical operation for expanding the airway, medical treatment (application of an alveolar surfactant preparation to the posterior region of the pharynx (see Published Japanese Translation of PCT Application No. 2001-507364 (hereinafter, referred to as the "Patent Document 3")), enhancement of the muscle groups by muscle strength stimulation exercise by low frequency vibration of the muscle groups of the tongue root in the cervical region (see Japanese Patent Application Laid-Open No. 2005-237807 (hereinafter, referred to as the "Patent Document 4")), etc. However, any of the approaches is a great burden for patients and the current state is that low-burden approaches to the prevention of the SAS have not been proposed.

Therefore, for the prevention of the SAS also, breathing exercise that patients can easily do is considered to be useful.
[Patent Document 1] Japanese Patent Application Laid-Open No. 62-277976
[Patent Document 2] Japanese Patent Application Laid-Open No. 2002-301047
[Patent Document 3] Published Japanese Translation of PCT Application No. 2001-507364
[Patent Document 4] Japanese Patent Application Laid-Open No. 2005-237807
[Non-Patent Document 1] Chacko N.Joseph, Cesare Porta, Gaia Casucci, Nadia Casiraghi, Mara Maffeis, Marco Rossi, Luciano Bernardi, "Slow Breathing Improves Arterial Baroreflex Sensitivity and Decreases Blood Pressure in Essential Hypertension", "Hypertension, October 2005", the American Heart Association, Volume 46, pp. 714-718

### DISCLOSURE OF THE INVENTION

### PROBLEMS TO BE SOLVED BY THE INVENTION

However, in both of the Patent Documents 1 and 2, the user needs to place a sensor on his/her abdomen or to put an instrument in his/her mouth. Hence, the user feels inconvenience or discomfort to handle them.

The present invention is made to solve problems such as those described above and an object of the present invention is therefore to provide a breathing exerciser and a breathing exercise program product that are capable of easily distinguishing a breathing state.

Alternatively, an object of the present invention is to provide a breathing exerciser and a breathing exercise program product that allow cost reduction.

### MEANS FOR SOLVING THE PROBLEMS

A breathing exerciser according to one aspect of the present invention comprises: a guide unit for guiding an exercise pattern of breathing to a user; a detecting unit capable of detecting an operation by the user; and a distinguishing unit for distinguishing during an exercise period during which the exercise pattern is guided, whether it is an exhalation state or an inhalation state, based on a signal from the detecting unit.

Preferably, the detecting unit includes an operation unit for accepting an operation by the user.
Preferably, the detecting unit includes an incline sensor capable of detecting a change in incline of a predetermined body part of the user from a reference position.

Preferably, the breathing exerciser further comprises: a calculating unit for calculating a breathing index representing a characteristic of a breathing state of the user, based on a result of the distinguishment by the distinguishing unit; a comparing unit for comparing the calculated breathing index with a predetermined reference value associated with the exercise pattern; and an alerting unit for alerting the user based on a result of the comparison by the comparing unit.

Note that it is preferable that the comparing unit compares whether the breathing index is within a predetermined range from the reference value and the alerting unit alerts the user when the breathing index is not within the predetermined range from the reference value.

Alternatively, it is preferable that the comparing unit compares whether the breathing index is within the predetermined range from the reference value and the alerting unit includes a counting unit for counting the number of cases where the breathing index is not within the predetermined range from the reference value; and a suspending unit for suspending the guiding of the exercise pattern by the guide unit when a result of the counting by the counting unit exceeds a predetermined threshold.

A breathing exercise program product according to another aspect of the present invention is a program for causing an arithmetic processing unit to perform a breathing exercise process in a control apparatus including an operation unit for accepting an operation by a user; an output unit for outputting sound or displaying an image; and the arithmetic processing unit, the breathing exercise program product causing to perform: a step of outputting a predetermined exercise pattern of breathing to the output unit; a step of receiving an operation signal from the operation unit during an exercise period during which the exercise pattern is outputted; and a step of distinguishing whether it is an exhalation state or an inhalation state, based on the received operation signal.

### EFFECT OF THE INVENTION

According to the present invention, without the need for a breathing sensor that detects breathing itself, a breathing state can be easily distinguished. Alternatively, since a breathing sensor is not required, cost reduction can be achieved.

### BRIEF DESCRIPTION OF THE DRAWINGS

FIG. 1 is a diagram showing a schematic view of a breathing exerciser in a first embodiment of the present invention.
FIG. 2 is a block diagram showing a configuration of the breathing exerciser in the first embodiment of the present invention.
FIG. 3A is a diagram showing a scene where a user is not pressing a state switch.
FIG. 3B is a diagram showing a scene where the user is pressing the state switch.
FIG. 4 is a flowchart showing a flow of a breathing exercise process in the first embodiment of the present invention.
FIG. 5 is a flowchart showing a length determination process in the first embodiment of the present invention.
FIG. 6 is a diagram showing an example of a screen to be displayed when inputting physical information.
FIG. 7 is a diagram showing an exemplary display of a breathing guide.
FIG. 8 is a diagram for describing a detailed exemplary display of the breathing guide based on an exercise pattern for during an exercise period.
FIG. 9 is a diagram showing a schematic view of a breathing exerciser in a second embodiment of the present invention.
FIG. 10 is a block diagram showing a configuration of an exerciser main body of the breathing exerciser in the second embodiment of the present invention.
FIG. 11 is a diagram showing a relationship between a use mode of an acceleration sensor and a read value by the acceleration sensor, in the second embodiment of the present invention.

### DESCRIPTION OF REFERENCE NUMERALS

1: exerciser main body, 2: acceleration sensor, 3: wiring line, 4: display unit, 5: driver, 10: I/O, 12: memory, 13: timer, 15a: drive apparatus, 15b: storage medium, 20: CPU, 21, 21A: operation unit, 24: speaker, 25: amplifier, 25: A/D converter, 100, 200: breathing exerciser, 120: pattern storage unit, 201: guide unit, 202: breathing state distinguishing unit, 203: index calculating unit, 204: comparing unit, 205: alerting unit, and 400: chair.

### BEST MODE FOR CARRYING OUT THE INVENTION

Embodiments of the present invention will be described in detail with reference to the drawings. Note that the same or corresponding parts are denoted by the same reference numerals throughout the drawings.

### [First Embodiment]

### (For configuration)

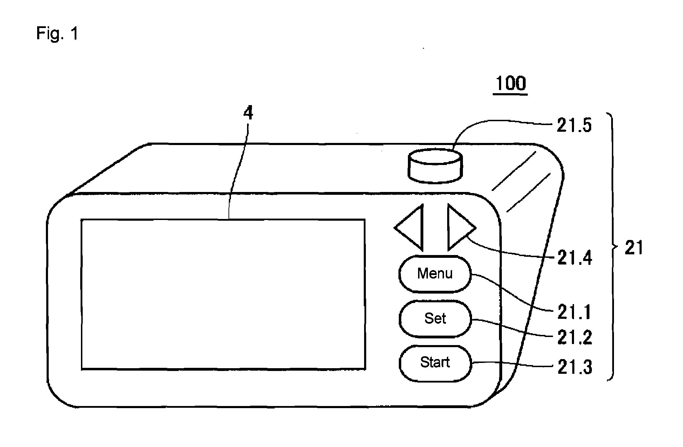

FIG. 1 is a diagram showing a schematic view of a breathing exerciser 100 in a first embodiment of the present invention.

Referring to FIG. 1, the breathing exerciser 100 according to the present embodiment includes a display unit 4 for displaying (outputting) characters, images, etc.; and an operation unit 21 for accepting an operation by a user. The display unit 4 is configured by, for example, an LCD (Liquid Crystal Display). The operation unit 21 includes a plurality of switches for accepting an input of an instruction from the user, e.g., a menu switch 21.1 for accepting an instruction to display a menu of functions of the breathing exerciser 100; a set switch 21.2 for accepting an instruction to set information displayed on the display unit 4; a start switch 21.3 for accepting an instruction to start exercise; left and right switches 21.4 for causing a cursor, etc., displayed on the display unit 4 to move left or right; and a switch (hereinafter, referred to as the "state switch") 21.5 which is capable of detecting an operation by the user and which is for accepting an operation corresponding to a breathing state.

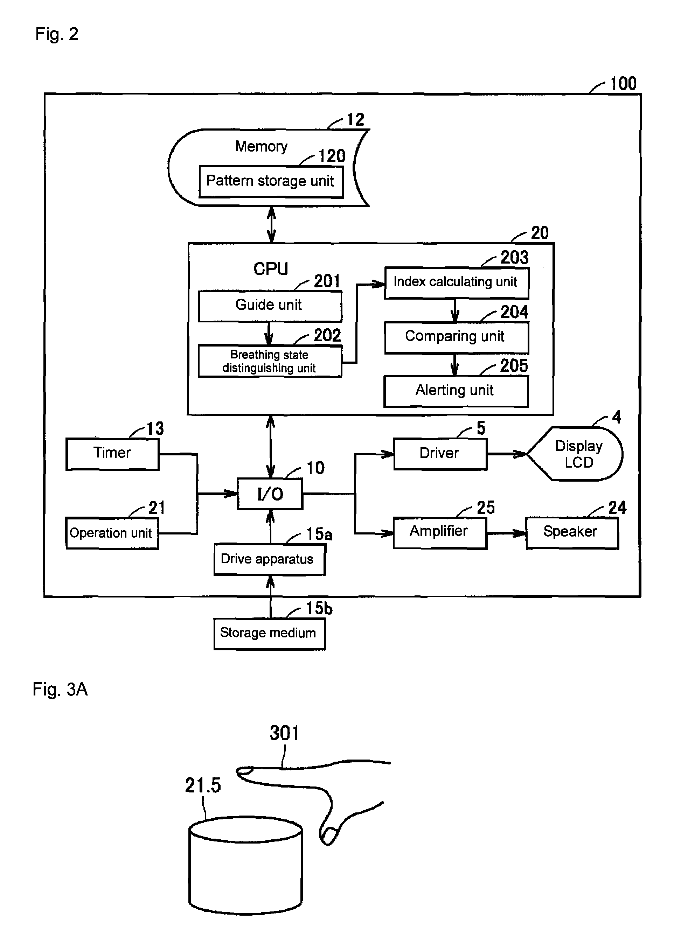

FIG. 2 is a block diagram showing a configuration of the breathing exerciser 100 in the first embodiment of the present invention. Referring to FIG. 2, the breathing exerciser 100 includes a CPU (Central Processing Unit) 20 for performing overall control of the breathing exerciser 100 and arithmetic operations; a memory 12 in which various data and programs are to be stored; a display unit 4; a driver 5 that controls the display operation of the display unit 4; an operation unit 21; a timer 13 that performs a timer operation to output timer data; an amplifier 25; a speaker 24 for outputting sound through the amplifier 25; and an input/output interface (I/O) 10 that controls input and output of data within the breathing exerciser 100. Timer data outputted from the timer 13 and information inputted to the operation unit 21 are inputted to the CPU 20 through the I/O 10. The CPU 20 controls the operations of the display unit 4 and the speaker 24 through the I/O 10.

Note that the breathing exerciser 100 may further include a drive apparatus 15a for reading various data and programs stored in a storage medium 15b. Note also that the breathing exerciser 100 does not necessarily need to include both the display unit 4 and the speaker 24 and may include at least one of them.

The memory 12 is, for example, a non-volatile memory (e.g., flash memory). The memory 12 includes a pattern storage unit 120 in which data on a plurality of exercise patterns are stored in advance. In the pattern storage unit 120, a plurality of exercise pattern data with different load levels are stored in advance.

The exercise pattern data includes information on at least exercise time (exercise period) or the number of breaths, a breathing cycle or exhalation time/inhalation time, and the depth of breathing. The load level is determined by, for example, at least one of parameters including at least exercise time or the number of breaths, a breathing cycle, and the depth of breathing.

Each exercise pattern data may include a plurality of pattern data with different load levels. Namely, a plurality of patterns with different parameters of at least one type (e.g., the depth of breathing) may be included in each exercise pattern.

The CPU 20 includes a guide unit 201 for guiding an exercise pattern of breathing to the user; a breathing state distinguishing unit 202 for distinguishing during an exercise period whether it is an exhalation state or an inhalation state, based on an operation signal from the state switch 21.5; an index calculating unit 203 for calculating a breathing index representing a characteristic of a user's breathing state, based on a result of the distinguishment by the breathing state distinguishing unit 202; a comparing unit 204 for comparing the breathing index calculated by the index calculating unit 203 with a predetermined reference value associated with an exercise pattern being guided (hereinafter, also referred to as a "guide pattern"); and an alerting unit 205 for alerting the user based on a result of the comparison by the comparing unit 204.

The "exercise period" is a period during which an exercise pattern is guided by the guide unit 201.
The guide unit 201 specifically performs a process of displaying information (hereinafter, also referred to as a "breathing guide") for guiding to breathing which is based on the exercise pattern (guide pattern), on the display unit 4. Note that although in the present embodiment description is made such that the exercise pattern is guided using the display unit 4, the exercise pattern may be guided by sound by the speaker 24. Note also that a determination method for a guide target exercise pattern will be described later.

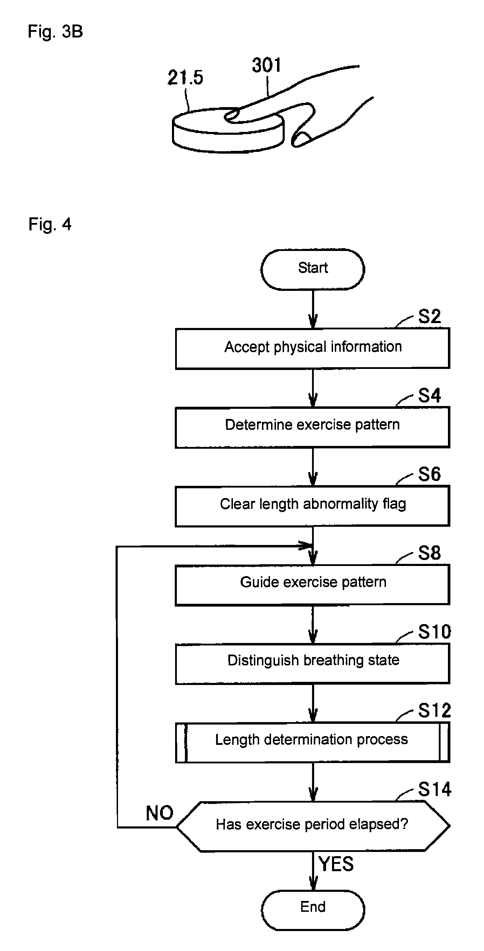

The breathing state distinguishing unit 202, for example, distinguishes that it is an exhalation state, when detecting the state switch 21.5 being pressed and distinguishes that it is an inhalation state, when the state switch 21.5 is not being pressed. Now, the use modes of the state switch 21.5 are shown in FIGS. 3A and 3B.

As shown in FIG. 3A, when the state switch 21.5 is not being pressed by a right hand 301 of the user, i.e., when the state switch 21.5 is OFF, the breathing state distinguishing unit 202 distinguishes that the user's breathing state is an inhalation state. On the other hand, as shown in FIG. 3B, when the state switch 21.5 is being pressed by the right hand 301 of the user, i.e., when the state switch 21.5 is ON, the breathing state distinguishing unit 202 distinguishes that the user's breathing state is an exhalation state. In this manner, the user performs, during the exercise period, an ON/OFF operation on the state switch 21.5 according to his/her breathing state. Therefore, for the disposition location of the state switch 21.5, it is desirable to dispose the state switch 21.5 at a location where the user can easily operate and the disposition location is not limited to the location shown in FIG. 1.

Also, a distinguishment method for a breathing state by the breathing state distinguishing unit 202 is not limited to an example such as that described above. For example, in an opposite manner, when the state switch 21.5 is OFF, it may be distinguished as an exhalation state, and when the state switch 21.5 is ON, it may be distinguished as an inhalation state.

Although in the present embodiment in order to accept an operation corresponding to a breathing state a dedicated switch (state switch 21.5) is provided, the present invention is not limited to such a configuration. Since the breathing state distinguishing unit 202 distinguishes a breathing state only during the exercise period, for example, the menu switch 21.1 may be allowed to have an equivalent function as the state switch 21.5 during the exercise period. Although in the present embodiment a switch is used as an example of an input apparatus for accepting an operation by the user, the input apparatus is not limited to the switch and can be any as long as at least two states (ON/OFF) can be detected. Alternatively, two switches to be operated in an exhalation state and an inhalation state, respectively, may be provided. In this case, the two switches may be respectively provided at locations where the switches can be operated by the left and right hands. Alternatively, the two switches may be respectively provided at locations where the switches can be operated by the left and right feet.

The index calculating unit 203 calculates, as a breathing index, for example, the length of one breathing cycle (breathing cycle), based on a distinguished breathing state and timer data from the timer 13. The breathing index is not limited to the breathing cycle and can be any as long as the breathing index is a value representing a characteristic of a user's breathing state. For example, the breathing index may be the number of breaths per predetermined period of time, exhalation time, inhalation time, a balance between an exhalation operation and an inhalation operation (e.g., a difference or ratio between exhalation time and inhalation time), etc.

The comparing unit 204 compares a breathing cycle calculated as the breathing index with a breathing cycle (reference value) associated with the guide pattern. When in the pattern storage unit 120 data on each exercise pattern does not include data on a breathing cycle, data on a breathing cycle may be stored in advance so as to be associated with data on each exercise pattern. Alternatively, a breathing cycle serving as a reference value may be derived (calculated) based on one or more data units included in data on each exercise pattern. The comparing unit 204 specifically compares whether the calculated breathing cycle is within a predetermined range from the reference (e.g., a range of 20% higher or lower than the reference value) with reference to a breathing cycle in the guide pattern. Alternatively, data in a range which is to be compared with the breathing index may be associated in advance with data on each exercise pattern. In this case, control for comparison can be simplified.

The alerting unit 205 specifically performs a process of outputting an alert sound from the speaker 24. Note that an alert is not limited to one by sound; for example, an alert message may be displayed on the display unit 4. The alerting unit 205 may include a process of counting the number of cases where the calculated breathing index is not within the predetermined range from the reference value, and suspending the exercise operation when a result of the counting exceeds a predetermined threshold.

Note that the operations of the functional blocks in the CPU 20 may be implemented by executing software stored in the memory 12 or at least one of them may be implemented by hardware.

### (For operation)

A breathing exercise process in the first embodiment of the present invention shown in a flowchart in FIG. 4 is stored in advance in the memory 12 as a program and a function of the breathing exercise process is implemented by the CPU 20 reading and executing this program. Note that the process shown below starts, for example, when the start switch 21.3 is pressed by the user.

Referring to FIG. 4, a flow of a processing operation will be described below. First, the CPU 20 accepts an input of physical information from the user (step S2). The physical information is information indicating a user's physical characteristic and includes at least one of, for example, a blood pressure value, height, weight, age, and sex. Here, it is assumed that information on height and weight is accepted as physical information.

Then, based on the accepted physical information, an exercise pattern (guide target exercise pattern) to be guided to the user in this breathing exercise process is determined (step S4). Specifically, for example, a process such as that shown below is performed. An association table in which height and weight are associated with identification information on an exercise pattern is stored in advance in, for example, the pattern storage unit 120. The CPU 20 identifies identification information associated with the user's height and weight in the association table. In this manner, an exercise pattern indicated by the identified identification information is determined as a guide target exercise pattern. The CPU 20 reads data on the guide target exercise pattern from the pattern storage unit 120. Note that the "guide target exercise pattern" here is the guide pattern mentioned previously, during an exercise period.

Next, the CPU 20 clears a "length abnormality flag" (step S6). The "length abnormality flag" refers to information for counting the number of cases where the breathing cycle is abnormal, i.e., the number of cases where the breathing cycle exceeds an allowable range (a predetermined range from the reference value). Note that this process should be performed before transitioning to an exercise period and thus may be performed, for example, before the process in step S2.

When the process in step S6 is completed, then, the guide unit 201 guides the exercise pattern determined in step S4 to the user (step S8). Specifically, based on the data on the exercise pattern read in step S4, a breathing guide (e.g., how much more exhalation and inhalation should be performed) is displayed on the display unit 4.

Subsequently, the breathing state distinguishing unit 202 distinguishes a user's breathing state based on a signal from the state switch 21.5 (step S10). It is desirable that a result of the distinguishment (e.g., 1: exhalation state or 0: inhalation state) is stored in, for example, an internal memory, which is not shown, along the time axis. A distinguishment method for a breathing state is as described above.

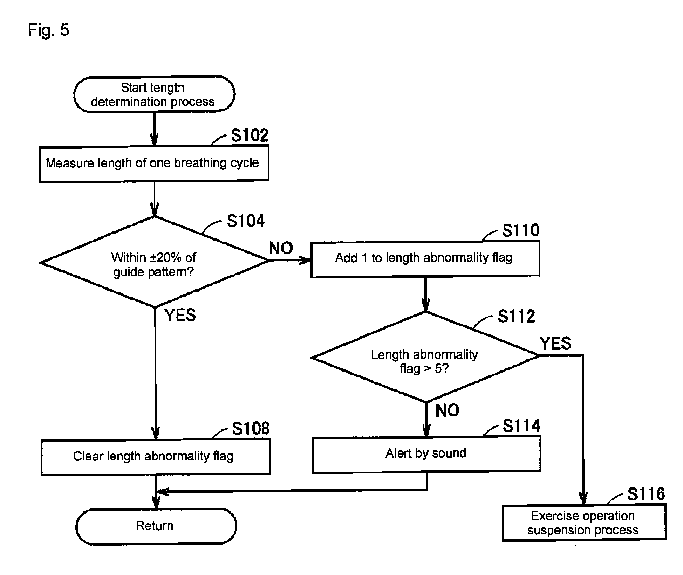

Subsequently, a "length determination process" which will be described later with reference to FIG. 5 is performed (step S12).

The processes in above-described steps S8 to S12 are repeated until the exercise period has elapsed (NO in step S14). If it is determined that the exercise period has elapsed (YES in step S14), then the breathing exercise process ends.

Note that information on a result of distinguishment of the breathing state in step S10 (e.g., a graph showing a transition of the breathing state) may be displayed on the display unit 4. Alternatively, the information on a result of distinguishment of the breathing state may be accumulated in, for example, a predetermined area of the memory 12 as an exercise result so that the information can be separately viewed.

FIG. 5 is a flowchart showing a length determination process in the first embodiment of the present invention.
Referring to FIG. 5, first, the index calculating unit 203 calculates, as a breathing index, the length of one breathing cycle (breathing cycle) based on a distinguishment result in step S10 (step S102).

Then, the index calculating unit 203 determines whether the calculated breathing cycle is within a range of 20% higher or lower than a breathing cycle associated with the guide pattern (within the allowable range) (step S104). If it is determined that the breathing cycle is within the allowable range (YES in step S104), then the length abnormality flag is cleared (step S108). When the process in step S108 is completed, processing is returned to the main routine.

On the other hand, if it is determined that the breathing cycle is outside the allowable range (NO in step S104), then the index calculating unit 203 adds 1 to the length abnormality flag (step S110). Subsequently, the comparing unit 204 determines whether the length abnormality flag exceeds a predetermined threshold (e.g., 5) (step S112). If it is determined that the flag is within the threshold (NO in step S112), then an alert by sound is issued by the speaker 24 (step S114). When the process in step S114 is completed, processing is returned to the main routine.

On the other hand, if it is determined in step S112 that the flag exceeds the threshold (YES in step S112), then an exercise operation suspension process is performed (step S116). Specifically, for example, first, an alert by sound is issued and the guiding of the exercise pattern by the guide unit 201 is suspended (stopped).

As described above, in the present embodiment, since the breathing state is distinguished by an ON/OFF operation of the state switch 21.5 by the user, a possibility arises where input error occurs. Therefore, in the present embodiment, when it is distinguished that the value of a breathing index (breathing cycle) is abnormal, the user is alerted to urge him/her to perform the right operation (operation according to his/her actual breathing state). Note that although here the exercise operation is suspended when the breathing index exceeds the allowable range for a predetermined number of times in a row, the exercise operation may be suspended when, during the exercise period, the breathing index exceeds the allowable range for the predetermined number of times (even not in a row).

Note that the calculation of a breathing index by the index calculating unit 203, the comparison by the comparing unit 204, and the alert by the alerting unit 205 are not always necessary in the above-described breathing exercise process. In this case, among the processes shown in the flowchart in FIG. 4, the processes in steps S6 and S12 are not necessary. Also, in this case, the CPU 20 shown in the block diagram in FIG. 2 does not need to include the index calculating unit 203, the comparing unit 204, and the alerting unit 205.

### (For exemplary display)

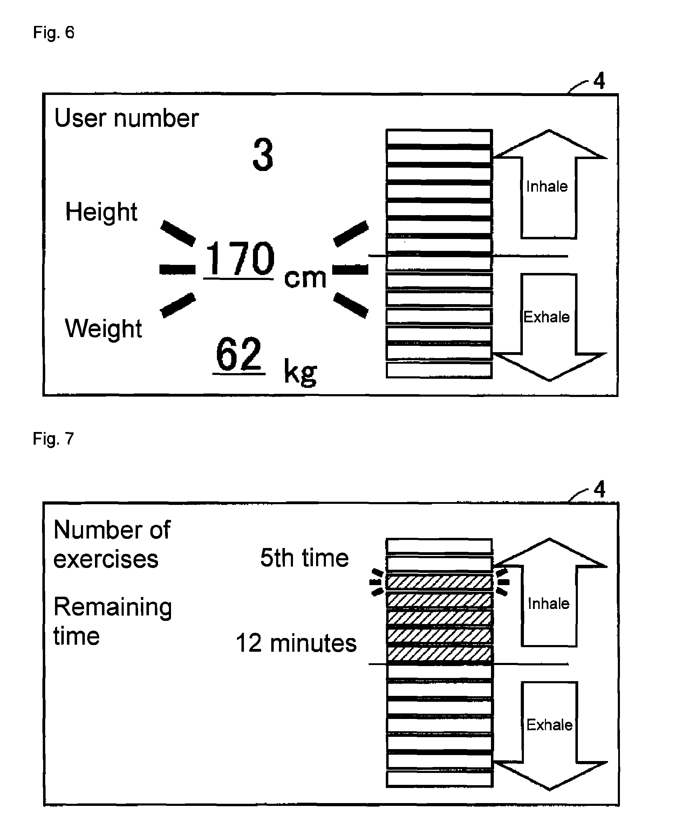

FIG. 6 is a diagram showing an example of a screen to be displayed when inputting physical information in step S2. As shown in FIG. 6, an item (height or weight) of physical information being inputted is displayed blinking. The physical information can be inputted using the left and right switches 21.4 and the set switch 21.2. Note that when exercise results are to be stored a user number may be inputted so that a plurality of users can use the exerciser. In this case, exercise results are stored so as to be associated with the inputted user number.

FIG. 7 is a diagram showing an exemplary display of a breathing guide in step S8. Referring to FIG. 7, a breathing guide is performed by highlighting/not highlighting 14 blocks displayed in a vertical direction on the screen. Also, the number of exercises (nth time) and remaining time are displayed in their respective predetermined areas.

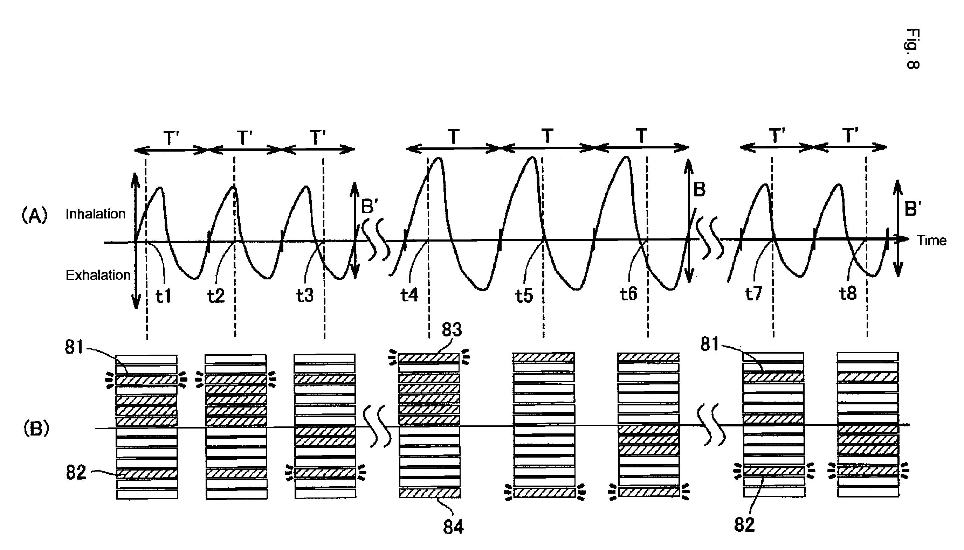

Detailed exemplary display of the breathing guide will be described with reference to FIG. 8.(A) of FIG. 8 is a diagram showing an example of an exercise pattern and (B) is a diagram showing an exemplary display of the breathing guide at arbitrary times t1 to t8 of the exercise pattern shown in (A).

Referring to (A) of FIG. 8, an exercise period based on the exercise pattern shown here includes a warm-up period, a substantial exercise period, and a cool-down period. That is, the exercise pattern includes a plurality of patterns.

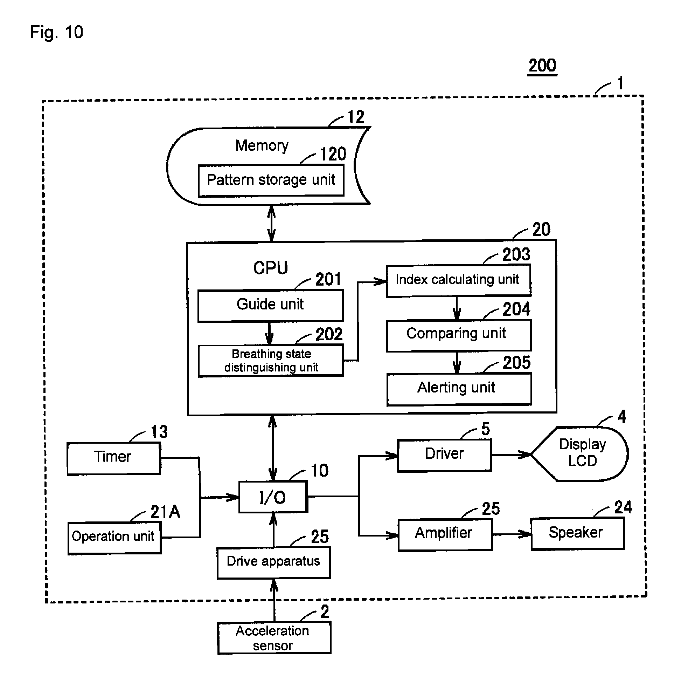

Referring to (B) of FIG. 10, in each breathing guide, a block at a location indicating the depth of breathing is fixedly displayed highlighted (displayed darkened). In the guides at times t1, t2, and t3 during a warm-up period, fifth blocks 81 and 82 respectively located upward and downward from the center are fixedly displayed highlighted. In the breathing guide at time t1, the first to third blocks located upward from the center are displayed highlighted and the block 81 is displayed blinking. By this, it is possible to inform the user of how much more time he/she should inhale. In the breathing guide at time t2, similarly, the block 81 is displayed blinking and all of the first to fourth blocks located upward from the center are displayed highlighted. By this, it is possible to inform that it is the end of an inhalation period.

At times t4, t5, and t6 during a substantial exercise period, seventh blocks (blocks at both ends) 83 and 84 respectively located upward and downward from the center are fixedly displayed highlighted. In the breathing guide at time t5, the block 84 is displayed blinking and blocks other than the block 83 are displayed not highlighted (displayed blanked). By this, the user is guided that inhalation is finished and thus the user should move to exhalation.

At times t7 and t8 during a cool-down period, as with the warm-up period, fifth blocks 81 and 82 respectively located upward and downward from the center are fixedly displayed highlighted. In the breathing guide at time t7, the block 82 is displayed blinking and the first block located upward from the center is displayed highlighted. By this, the user is guided that an exhalation state should be continued for on the order of another one-half.

Note that the display mode of the breathing guide is not limited to highlight/no highlight such as that described above; for example, the display color of blocks can be changed.

A breathing exercise method to be performed by the breathing exerciser 100 of the present invention, such as that descried above, can also be provided in the form of a program. Such a program can also be provided in the form of a program product by storing the program on an optical medium, such as a CD-ROM (Compact Disk-ROM), or in the computer-readable storage medium 15b, such as a memory card. Alternatively, the program can also be provided by download via a network.

A program product to be provided is executed by, for example, being installed in the memory 12. Alternatively, the program product may be executed by being installed in a program storage unit such as hard disk which is not shown. Note that the program product includes a program itself and a storage medium storing the program.

Even in a control apparatus including hardware similar to that of the breathing exerciser 100 shown in FIG. 2, the above-described breathing exercise process can be implemented. That is, the control apparatus should include at least an operation unit for accepting an operation by the user; an output unit for outputting sound or displaying an image; and an arithmetic processing unit such as a CPU. Then, by the arithmetic processing unit reading and executing a program for a breathing exercise process, the control apparatus can function as a breathing exerciser. Such a control apparatus may be, for example, a general personal computer, mobile phone, game machine, or the like.

### [Second Embodiment]

Next, a second embodiment of the present invention will be described. A breathing exerciser in the second embodiment is different from the breathing exerciser 100 in the first embodiment in part of hardware configuration. The main difference from the first embodiment will be described below.

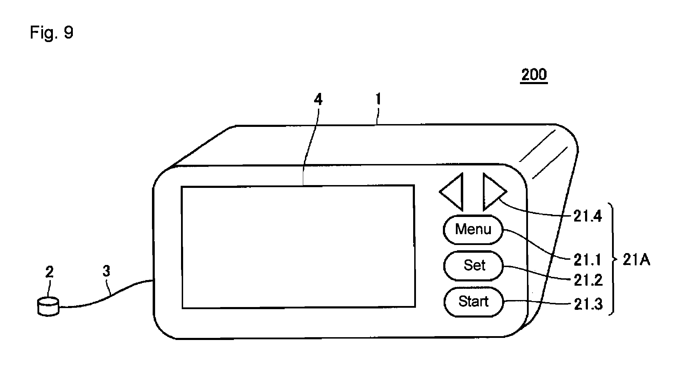

FIG. 9 is a diagram showing a schematic view of a breathing exerciser 200 in the second embodiment of the present invention.
Comparing with the breathing exerciser 100 in the first embodiment, in the breathing exerciser 200, a state switch 21.5 for accepting an operation corresponding to a breathing state is not included in an operation unit 21A. Instead of this, the breathing exerciser 200 includes an incline sensor capable of detecting an operation by the user and capable of detecting a change in the incline of a predetermined body part of the user from a reference position, e.g., an acceleration sensor 2; and a wiring line 3 for supplying a signal from the acceleration sensor 2 into an exerciser main body 1.

FIG. 10 is a block diagram showing a configuration of the breathing exerciser 200 in the second embodiment of the present invention. Comparing with the breathing exerciser 100 in the first embodiment, the breathing exerciser 200 includes, instead of the drive apparatus 15a, an A/D converter 25 for converting an output signal from the acceleration sensor 2 from an analog signal to a digital signal. The signal converted by the A/D converter 25 is inputted to a CPU 20 through an I/O 10.

In the second embodiment, a breathing state distinguishing unit 202 distinguishes a user's breathing state based on a signal from the acceleration sensor 2.

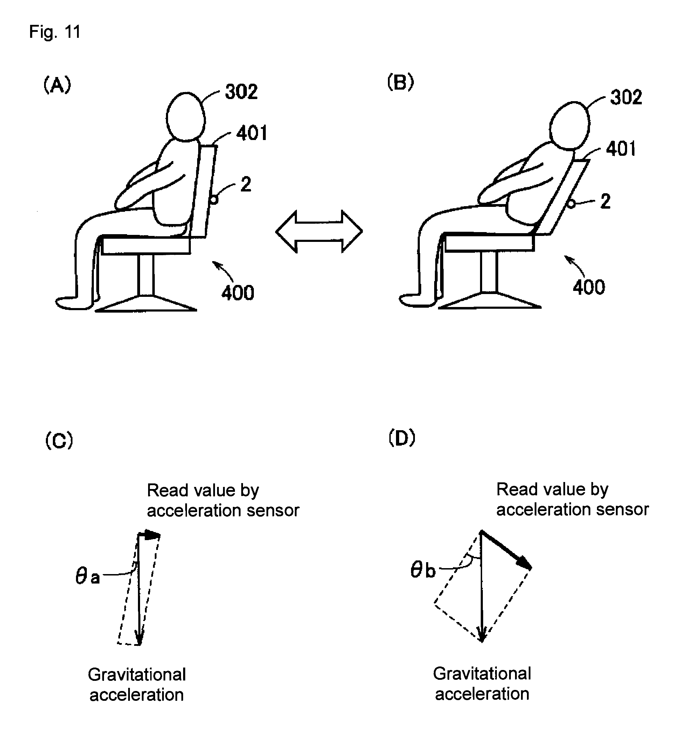

(A) of FIG. 11 is a diagram showing a state in which the user has just begun to incline his/her back in a rear direction and (B) is a diagram showing a state in which the user is in the process of inclining his/her back in the rear direction. (C) is a diagram showing a read value by the acceleration sensor 2 in the state (A), and (D) is a diagram showing a read value by the acceleration sensor 2 in the state (B).

(A) and (B) of FIG. 11 show an example in which the acceleration sensor 2 is attached to a backrest 401 of a chair 400. The chair 400 includes a seating portion where a user 302 is seated; and the backrest 401. The backrest 401 is formed to be able to be inclined in the rear direction and to be able to return to a predetermined position in a state (normal state) in which the user 302 is not seated.

During an exercise period, the user 302 performs, in conjunction with a breathing operation, an operation of, for example, pressing the backrest 401 upon an inhalation state and returning to the predetermined position by using the resilience of the backrest 401 upon an exhalation state. That is, the user 302 repeats postures shown in (A) and (B) of FIG. 11 during the exercise period.

It is desirable that the acceleration sensor 2 is able to detect the angle (θ) of incline of the backrest 401 at the reference position. In the present embodiment, the acceleration sensor 2 can calculate the angle by measuring a change in gravitational acceleration. In (C) of FIG. 11 an angle θa is calculated, and in (D) of FIG. 11 an angle θb is calculated. Namely, in the present embodiment, the reference position is a vertical direction and the acceleration sensor 2 can detect a change in the incline in the vertical direction of the backrest 401 that can be operated with being in close contact with the back of the user 302.

The breathing state distinguishing unit 202 can distinguish whether it is an inhalation state or an exhalation state, based on output data (information on incline angle) from the acceleration sensor 2. The breathing state distinguishing unit 202 obtains information on incline angle, for example, every 0.1 seconds to determine angle change conditions (whether the angle is changed in an increasing direction or changed in a decreasing direction). Then, when it is determined that the incline angle is changed in the increasing direction, the breathing state distinguishing unit 202 distinguishes that it is an exhalation state and when it is determined that the incline angle is changed in the decreasing direction, the breathing state distinguishing unit 202 distinguishes that it is an inhalation state.

As such, in the second embodiment too, the breathing exerciser 200 can distinguish a user's breathing state without including a breathing sensor. Also, in the second embodiment too, the breathing exercise process shown in FIG. 4 can be implemented, where only a distinguishment method for a breathing state is different from that for the first embodiment.

Although in the second embodiment the acceleration sensor 2 is provided on the rear side of the backrest 401 of the chair, the acceleration sensor 2 may be provided on the inner side (the side opposed to the rear) of the backrest 401. Alternatively, without using the chair 400, the acceleration sensor 2 may be directly attached to the back of the user 302 to detect a change in the incline of the back of the user 302 relative to the reference position.

Alternatively, the body part which is the detection target of incline change is not limited to the back and may be, for example, the arm or leg of the user.

Although in the present embodiment description is made such that the reference position is a vertical direction, the reference position is not limited thereto. For example, the reference position may be a position indicating the incline of the back in a state in which the user is first seated or may be set by the user.

Note that a breathing exerciser can also be provided that includes a chair 400 and an exerciser main body 1, in which an acceleration sensor 2 is included in advance in the chair 400.

As described above, since the breathing exercisers in the first and second embodiments of the present invention do not include a breathing sensor, the configuration of the breathing exercisers can be simplified. Also, because of this, the cumbersomeness of maintenance of the breathing sensor can be eliminated.

The embodiments disclosed herein are to be considered in all respects as illustrative and not restrictive. The scope of the present invention is indicated by the appended claims rather than by the foregoing description, and all changes that come within the meaning and range of equivalency of the appended claims are intended to be embraced therein.

## Claims

1. A breathing exerciser comprising:
a guide unit (201) for guiding an exercise pattern of breathing to a user;
a detecting unit (21, 20) capable of detecting an operation by the user; and
a distinguishing unit (202) for distinguishing during an exercise period during which the exercise pattern is guided, whether it is an exhalation state or an inhalation state, based on a signal from the detecting unit.

2. The breathing exerciser according to claim 1, wherein the detecting unit includes an operation unit (21) for accepting an operation by the user.

3. The breathing exerciser according to claim 1, wherein the detecting unit includes an incline sensor (2) capable of detecting a change in incline of a predetermined body part of the user from a reference position.

4. The breathing exerciser according to claim 1 further comprising:
a calculating unit (203) for calculating a breathing index representing a characteristic of a breathing state of the user, based on a result of the distinguishment by the distinguishing unit;
a comparing unit (204) for comparing the calculated breathing index with a predetermined reference value associated with the exercise pattern; and
an alerting unit (205) for alerting the user based on a result of the comparison by the comparing unit.

5. A breathing exercise program product, a program being for causing an arithmetic processing unit to perform a breathing exercise process in a control apparatus including an operation unit for accepting an operation by a user; an output unit for outputting sound or displaying an image; and the arithmetic processing unit, the breathing exercise program product causing to perform:
a step (S8) of outputting a predetermined exercise pattern of breathing to the output unit;
a step (S10) of receiving an operation signal from the operation unit during an exercise period during which the exercise pattern is outputted; and
a step (S10) of distinguishing whether it is an exhalation state or an inhalation state, based on the received operation signal.
